# EUROPÄISCHE PATENTANMELDUNG

(11) **EP 0 983 987 A1**
(43) Veröffentlichungstag der Anmeldung: **08.03.2000**
(21) Anmeldenummer: 99116088.8
(22) Anmeldetag: 17.08.1999
(51) Int. Cl.: C07C 45/29

(54) **Verfahren zur Herstellung von alpha-Diketonen aus Ketolen oder Ketalen von Ketolen**

(30) Priorität: 21.08.1998 DE 19838046
(71) Anmelder: BASF AKTIENGESELLSCHAFT, 67056 Ludwigshafen (DE)
(72) Erfinder: Aquila, Werner, Dr., 68309 Mannheim (DE); Botzem, Jörg, Dr., 67117 Limburgerhof (DE); Brunner, Melanie, Dr., 67105 Schifferstadt (DE); Fuchs, Hartwig, 67063 Ludwigshafen (DE); Krause, Wolfgang, Dr., 68782 Brühl (DE); Plandl, Klaus, Dr., 76707 Hambrücken (DE); Schäfer-Lüderssen, Ulrich, Dr., 67063 Ludwigshafen (DE)

(57) **Zusammenfassung**

Verfahren zur Herstellung von Carbonylverbindungen der allgemeinen Formel I 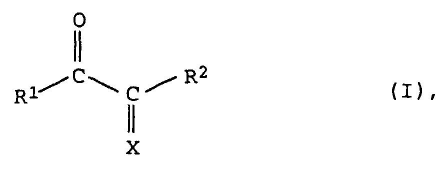 in der R¹ und R² für einen Kohlenwasserstoffrest stehen, oder aber
R¹ und R² zusammen für eine gegebenenfalls substituierte Alkylengruppe steht,
und x für = O oder 2 Alkoxygruppen steht, das dadurch gekennzeichnet ist, daß man Alkohole der allgemeinen Formel II 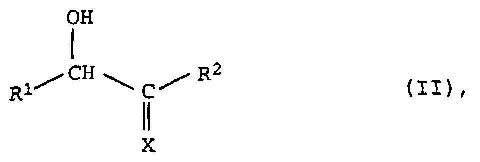
in der R¹ bis R² und X die oben angegebene Bedeutung haben,
mit Sauerstoff in der Gasphase
   a) bei Temperaturen von 270 bis 600°C an Silber-Schalenkatalysatoren, die eine abriebfeste Schale von metallischem Silber auf einem Kern aus inertem Trägermaterial enthalten, oder aber
   b) bei Temperaturen von 450 bis 750°C an Silberkristallen und/oder Kupferkristallen mit einer Korngröße von 0,1 bis 2,5 mm während einer Verweilzeit von maximal 0,1 Sekunden oxidiert.

Beansprucht wird auch ein vorteilhaftes Gesamtverfahren zur Herstellung von α-Diketonen, vorzugsweise Diacetyl, aus dem entsprechenden Keton, insbesondere Methylethylketon, über die Carbonylverbindungen der allgemeinen Formel I, in der X für zwei Alkoxygruppen steht.

## Beschreibung

Die Erfindung betrifft ein verbessertes Verfahren zur Herstellung von Ketonen der allgemeinen Formel I 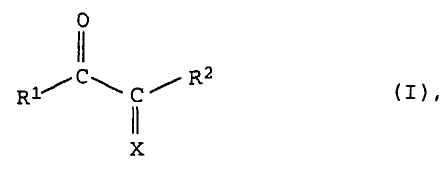 in der R¹ und R² für einen Kohlenwasserstoffrest stehen, oder aber
R¹ und R² zusammen für eine gegebenenfalls substituierte Alkylengruppe stehen
und X für = O oder 2 Alkoxygruppen steht, durch katalytische Oxidation der entsprechenden Alkohole der allgemeinen Formel II 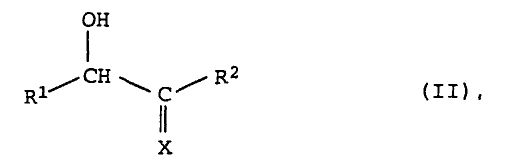
und gegebenenfalls anschließende Hydrolyse der erhaltenen α-Diketonmonoketale, insbesondere eine technisch vorteilhafte Herstellung von Diacetyl.

Die wichtigsten Vertreter der Ketone der allgemeinen Formel I sind - wegen ihrer besonderen olfaktorischen Eigenschaften - das 2,3-Butan-dion (Diacetyl), 1,2-Cyclopentan-dion, 1,2-Cyclohexan-dion, 2,3-Pentan-dion, 2,3-Hexan-dion und 3,4-Hexan-dion sowie die Monoketale der genannten 1,2-Cycloalkan-dione, insbesondere aber das Diacetyl.

Wegen der großen Nachfrage nach diesen Verbindungen, insbesondere dem Diacetyl, hat es daher nicht an Versuchen gefehlt, geeignete Herstellungsverfahren für diese Diketone zu finden. So ist aus US 2,043,950 (1936) ein Verfahren zur katalytischen Oxidation von Ketolen bekannt, bei dem Ketole in Gegenwart fester Oxidationskatalysatoren bei erhöhter Temperatur mit molekularem Sauerstoff umgesetzt werden. Als Beispiele für feste Oxidationskatalysatoren werden Metalle, Metallegierungen, Metallsalze und Metalloxide, vorzugsweise Elemente der 3. Periode des Periodensystems der Elemente, wie Ti, V, Cr, Mn, Fe, Co, Ni, Cu, Zn und Se, insbesondere Cu, CuO und Ag genannt. Gemäß den Beispielen wird 2,3-Butandion (Diacetyl) aus 3-Hydroxy-2-butanon (Acetoin) an CuO in Ausbeuten von nur 48 %, bezogen auf umgesetztes Acetoin, und an aktiviertem Kupfer je nach Umsatz in Ausbeuten von 43 bis 82 % der Theorie erhalten. Nachteilig an diesem Verfahren ist, daß selbst bei Verwendung der bevorzugten Katalysatoren gute Ausbeuten, z.B. von 82 %, nur bei geringen Umsätzen (25%) erhalten werden, während bei guten Umsätzen (z.B, 77 %) nur niedrige Ausbeuten (z.B. 43 %) erhalten werden.

Aus dem Derwent-Referat von SU 825 489 (1979) ist ein Verfahren bekannt, bei dem Diacetyl durch Erhitzen von Acetoin in wäßriger Lösung mit Eisen(III)chlorid in 75 %iger Ausbeute erhalten wird. Nachteilig an diesem Verfahren sind die unzureichenden Ausbeuten und die in technischem Maßstab aufwendige Verfahrensführung.

Weiterhin ist aus DE-OS 28 31 229 (1978) ein Verfahren zur Herstellung von Diketonen bekannt, bei dem man Glykole in Gegenwart eines Katalysators bestehend aus zwei oder mehr Schichten an Silber- und/oder Kupferkristallen bestimmter Korngröße und unter bestimmten Bedingungen der Temperatur und Katalysatorzusammensetzung mit Sauerstoff oxidiert. Nachteilig an diesem Verfahren ist zum einen, daß die Herstellung des als Ausgangsverbindung für Diacetyl benötigte 2,3-Butandiol sehr aufwendig und dementsprechend das 2,3-Butandiol recht teuer ist, und zum anderen, daß bei der Umsetzung von 2,3-Butandiol zu Diacetyl gemäß Beispiel 3 dieses Patents nur Ausbeuten von 76 % der Theorie erhalten werden.

Weiterhin ist aus DE-OS 28 31 595 (1978) ein Verfahren zur Herstellung von speziellen Carbonylverbindungen durch Oxidation der entsprechenden Alkohole mit Sauerstoff in Gegenwart von Silberkristallen und/oder Kupferkristallen mit einer Korngröße von 0,01 Mikrometer bis 2,5 Millimeter bei Temperaturen von 450 bis 700°C und einer Verweilzeit von maximal 0,1 sekunden bekannt. Gemäß diesem Verfahren werden im wesentlichen aliphatische oder cycloaliphatische Aldehyde oder Cycloalkanone hergestellt. Es wird aber auch die Möglichkeit beschrieben, mit diesem Verfahren α-Ketocarbonsäureester herzustellen. Bei dem einzigen diesbezüglichen Beispiel wird α-Oxo-isocapronsäure-isobutylester jedoch nur in Ausbeuten von 81 % der Theorie erhalten.

Aus DD 296 274 A (1990) ist ein Verfahren zur Herstellung von α-Diketonen bekannt, bei dem α-Acetoxyalkine in einer Carbonsäure als Lösungsmittel bei 0 bis 100°C in Gegenwart von einem Pd(II)-Salz oxidiert werden und die erhaltenen α,β-ungesättigten α-Acetoxyketone durch anschließende Hydrolyse in α-Diketone überführt werden. Nachteilig an diesem Verfahren sind die unbefriedigenden Ausbeuten sowie die technisch aufwendige Verfahrensführung.

Weiterhin ist aus C.A. 123:285011X (Referat zu Stud. Surf. Sci. Catal. 1994, 82, Seiten 853-60) bekannt, Diacetyl durch Partialoxidation von Methylethylketon an einem Vanadiumoxidkatalysator herzustellen. Nachteilig an diesem Verfahren ist, daß als Nebenprodukte größere Mengen an Essigsäure, Acetaldehyd, Methylvinylketon, Propionaldehyd und CO₂ gebildet werden.

Bei neueren Versuchen zur Oxidation von Acetoin mit Ce(IV) in wäßriger HClO₄ konnte überhaupt kein Diacetyl isoliert werden, da diese zu Essigsäure weiterreagierte (vgl. Acta Chem. Scand. (1991), 45(5), Seiten 543-5).

Weiterhin ist aus WO 96/04229-A1 bekannt, Diole mit gasförmigem Fluor zu 1,2-Hydroxy-carbonylverbindungen oder zu 1,2-Dionen zu oxidieren. Nachteilig an diesem Verfahren ist, daß Fluor ein relativ teures und in der Handhabung gefährliches Oxidationsmittel ist.

In EP 658 533 (1994) ist die Oxidation von Diolen mit o-Iodoxybenzoesäure in Dimethylsulfoxid bei 40 bis 50°C beschrieben. Nachteilig an diesem Verfahren ist, daß das Oxidationsmittel aufwendig herstellbar und daher recht teuer ist.

Weiterhin ist aus EP 430 406 die fermentative Herstellung von Diacetyl und Acetoin aus Zuckern mit Hilfe von Milchsäurebakterien bekannt. Nachteilig an diesem Verfahren ist, daß die erzielbaren Ausbeuten noch unzureichend sind.

Es war daher die Aufgabe der Erfindung, ein möglichst vorteilhaftes Verfahren zur Herstellung von α-Diketonen aus den entsprechenden Ketolen oder Ketalen dieser Ketole zu entwickeln, das es erlaubt, α-Diketone, insbesondere das Diacetyl, auch in industriellem Maßstab in sehr guten Ausbeuten und Raumzeitausbeuten aus relativ gut zugänglichen Ausgangsverbindungen, mit preiswerten Oxidationsmitteln sowie an lange Zeit stabilen Katalysatoren sowie mit guten sensorischen Eigenschaften herzustellen.

Als verhältnismäßig gut zugängliche Ausgangsverbindungen haben sich Ketole und Ketale von Ketolen erwiesen. Für die Herstellung von dem als Butteraroma besonders begehrten Diacetyl sind das das Acetoin, das durch elektrochemische Oxidation von 2-Butanon gut zugänglich ist, und insbesondere 3,3-Dialkoxy-2-butanole, die gemäß dem in EP 460 451 beschriebenen Verfahren durch elektrochemische Oxidation von Methylethylketon (2-Butanon) in Gegenwart von Alkanolen, Wasser und einem Hilfselektrolyten auch technisch recht gut zugänglich sind.

Gegenstand der Erfindung ist daher ein Verfahren zur Herstellung von Carbonylverbindungen der allgemeinen Formel I 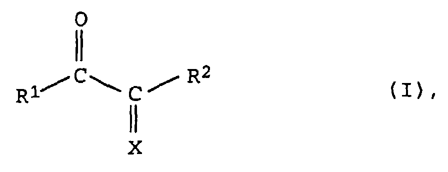 in der R¹ und R² für einen gesättigten oder ungesättigten, verzweigten oder unverzweigten aliphatischen, cycloaliphatischen, aromatischaliphatischen oder cycloaliphatischaliphatischen Rest mit 1 bis 10 C-Atomen, vorzugsweise eine Alkylgruppe mit 1 bis 4 C-Atomen, insbesondere eine Methylgruppe oder Ethylgruppe, stehen, oder aber
R¹ und R² zusammen für eine gegebenenfalls durch niedere Alkylgruppen substituierte Alkylengruppe mit 3 bis 10 C-Atomen, vorzugsweise eine Propylen- oder Butylengruppe, steht,
und x für = O oder 2 Alkoxy-gruppen -OR³ steht, wobei R³ für einen gesättigten oder ungesättigten, verzweigten oder unverzweigten aliphatischen Rest mit 1 bis 6 C-Atomen, vorzugsweise für eine Alkylgruppe mit 1 bis 4 C-Atomen, insbesondere eine Methylgruppe oder Ethylgruppe steht, oder aber die beiden R³ zusammen für eine gegebenenfalls durch niedere Alkylgruppen, insbesondere durch Methyl- oder Ethylgruppen, substituierte Alkylengruppe mit 3 bis 6 C-Atomen stehen,
das dadurch gekennzeichnet ist, daß man Alkohole der allgemeinen Formel II 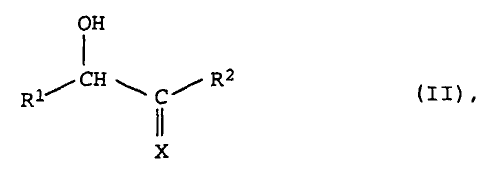 in der R¹ bis R³ und X die oben angegebene Bedeutung haben, mit Sauerstoff in der Gasphase
a) bei Temperaturen von 270 bis 600°C, vorzugsweise 300 bis 550°C, an Silberschalenkatalysatoren, die eine abriebfeste Schale von metallischem Silber auf einem Kern aus inertem Trägermaterial enthalten, oder aber
b) bei Temperaturen von 450 bis 750°C, vorzugsweise von 480 bis 600°C, an Silberkristallen und/oder Kupferkristallen, vorzugsweise an Silberkristallen, mit einer Korngröße von 0,1 bis 2,5 mm während einer Verweilzeit von maximal 0,1 Sekunden oxidiert.

Als Ausgangsstoffe der allgemeinen Formel II für das erfindungsgemäße Verfahren kann man also Verbindungen, in denen X für = 0 steht, also Ketole, verwenden. Ketole sind im allgemeinen technisch relativ gut durch elektrochemische Oxidation von Ketonen zugänglich. Genannt seien beispielsweise das 3-Hydroxy-2-butanon (Acetoin), 2-Hydroxy-1-cyclopentanon, 2-Hydroxy-1-cyclohexanon, 2-Hydroxy-3-pentanon, 3-Hydroxy-2-pentanon, 3-Hydroxy-4-hexanon und 2-Hydroxy-3-hexanon, insbesondere das Acetoin.

Als Ausgangsstoffe der allgemeinen Formel II kann man mit Vorteil auch Verbindungen, in denen X für 2 Alkoxygruppen steht, verwenden. Diese erhält man auch technisch sehr vorteilhaft gemäß dem in EP 460 451 B1 beschriebenen elektrochemischen Verfahren. So erhält man beispielsweise das für die Herstellung von Diacetyl besonders geeignete 3,3-Dimethoxy-2-butanol nach diesem Verfahren aus 2-Butanon (Methyl-ethyl-keton) in einer Selektivität von 70 % der Theorie (vgl. Beispiel 1A). Mit Hilfe des erfindungsgemäßen Verfahrens kann hieraus das 3,3-Dimethoxy-2-butanon in Ausbeuten von über 90 % gewonnen werden (vgl. Beispiel 1B), welches durch alkalische oder besser saure Hydrolyse in an sich bekannter Weise in praktisch quantitativer Ausbeute in Diacetyl überführt werden kann. So ergibt sich ein sehr vorteilhaftes Gesamtverfahren zur Herstellung von Diacetyl aus dem gut zugänglichen Methylethylketon.

Gegenstand der Erfindung ist daher auch ein wie oben definiertes Verfahren, das dadurch gekennzeichnet ist, daß man die bei der Oxidation von Alkoholen der allgemeinen Formel II, in der X für zwei Alkoxy-Gruppen steht, erhaltenen Carbonylverbindungen der allgemeinen Formel I, in der X für zwei Alkoxygruppen steht, anschließend in an sich bekannter Weise in Carbonylverbindungen der allgemeinen Formel I, in der X für = O steht, hydrolysiert, sowie ein solches Verfahren, das dadurch gekennzeichnet ist, daß man als Ausgangsverbindungen Alkohole der allgemeinen Formel II, in der X für zwei Alkoxygruppen steht, verwendet, die durch elektrochemische Oxidation von Ketonen der allgemeinen Formel III 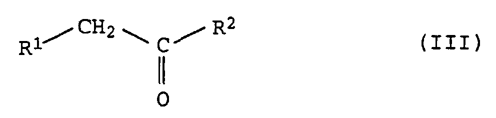 in Gegenwart von C₁- bis C₄-Alkanolen, Wasser und Hilfselektrolyten erhalten worden sind, verwendet.

Gegenstand der Erfindung ist auch ein Verfahren zur Herstellung von Carbonylverbindungen der allgemeinen Formel I 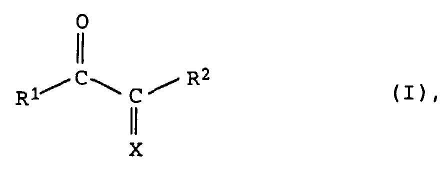 in der R¹ und R² für eine Methylgruppe stehen, oder aber R¹ und R² zusammen für eine ggf. durch niedere Alkylgruppen substituierte Alkylengruppe mit 3 bis 10 C-Atomen steht,
und X für = O steht, das dadurch gekennzeichnet ist, daß man
A. Alkohole der allgemeinen Formel II 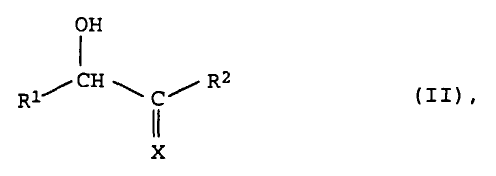
   in der R¹ und R² die oben angegebene Bedeutung haben, und
   X für zwei Alkoxy-gruppen -OR³ steht, wobei R³ eine Alkylgruppe mit 1 bis 4 C-Atomen bedeutet, oder aber die beiden R³ zusammen für eine gegebenenfalls durch niedere Alkylgruppen substituierte Alkylengruppe mit 3 bis 6 C-Atomen stehen, verwendet, die
      durch elektrochemische Oxidation von Ketonen der allgemeinen Formel III 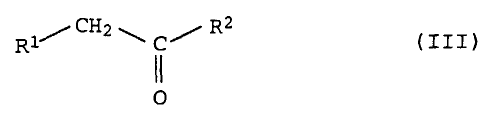 in Gegenwart von C₁- bis C₄-Alkanolen, Wasser und Hilfselektrolyten hergestellt worden sind,
B. diese mit Sauerstoff in der Gasphase
   a) bei Temperaturen von 270 bis 600°C, vorzugsweise 300 bis 550°C, in einem Rohrreaktor oder Rohrbündelreaktor an Silber-Schalenkatalysatoren, die eine abriebfeste Schale von metallischem Silber auf einem Kern aus inertem Trägermaterial enthalten, oder aber
   b) bei Temperaturen von 450 bis 750°C, vorzugsweise 480 bis 600°C, an Silberkristallen mit einer Korngröße von 0,1 bis 2,5 mm während einer Verweilzeit von maximal 0,1 Sekunden oxidiert und
C. die dabei erhaltenen Carbonylverbindungen der allgemeinen Formel I, in der X für zwei Alkoxygruppen steht, in an sich bekannter Weise in Carbonylverbindungen der allgemeinen Formel I, in der X für = O steht, hydrolysiert.

### Zu Variante a) des erfindungsgemäßen Verfahrens

Als Silberschalenkatalysatoren, die eine abriebfeste Schale von metallischem Silber auf einem Kern aus inertem Trägermaterial enthalten, verwendet man Katalysatoren, die auf einfache Weise durch Beschichten von inertem Trägermaterial, wie α-Aluminiumoxid, Siliciumcarbid oder Steatit mit Silber nach an sich bekannten Verfahren, wie Flammspritzen, Plasmaspritzen oder Aufdampfen, hergestellt werden. Besonders geeignete Schalenkatalysatoren bestehen z.B. aus einem Träger aus Steatit, der z.B. die Form von Kugeln, Ringen oder Halbringen aufweist, mit einer darauf aufgebrachten katalytischen Masse aus Silber, die z.B. 0,1 bis 10 Gew.-%, bezogen auf den fertigen Schalenkatalysator, ausmacht.

Mit Vorteil verwendet man Silberschalenkatalysatoren, die elementares Silber auf Inertmaterial in Form von Kugeln oder vergleichbaren geometrischen Formen enthalten, insbesondere solche, die elementares Silber auf Steatit-Kügelchen enthalten. Der Durchmesser der Katalysatorkugeln kann im Bereich von 1 bis 10 mm, vorzugsweise 1 bis 5 mm, liegen.

Als besonders vorteilhaftes Reaktionsgefäß für diese Verfahrensvariante haben sich einzelne Reaktionsrohre oder Reaktionsrohrbündel erwiesen, die mit den festen Katalysatorteilchen gefüllt sind. Mit Vorteil verwendet man Reaktionsrohre, die einen Innendurchmesser von nur 0,5 bis 30 mm, vorzugsweise 10 bis 20 mm, aufweisen. Durch diese Rohre wird das Gemisch aus dem verdampften Alkohol der Formel II und Sauerstoff oder vorzugsweise einem Sauerstoff enthaltenden Gas, insbesondere Luft, geleitet. Sie werden von einem Medium umflossen, das am Anfang die für das Starten der Reaktion notwendige Energie liefert und im Verlauf der Reaktion die bei der exothermen Reaktion gebildete Reaktionswärme abführt.

Die Länge der Reaktionsrohre beträgt im allgemeinen mindestens 5 cm, vorzugsweise 10 bis 60 cm.

Die Größe der Katalysatorpartikel muß dem Durchmesser der verwendeten Reaktionsrohre angepaßt sein. Die Durchmesser der Katalysatorpartikel liegen im allgemeinen im Bereich von 1:10 bis 1:5 im Verhältnis zum Innendurchmesser der Reaktionsrohre.

Die Aufarbeitung des den Reaktor verlassenden gasförmigen Reaktionsgemisches erfolgt auf übliche Weise. Beispielsweise absorbiert man die heißen Reaktionsgase unmittelbar nach Austritt aus dein Reaktor mit einem Lösungsmittel oder vorzugsweise im kondensierten Produktgemisch. Die Verweilzeit des Gasgemisches im Reaktionsrohr beträgt nur etwa 0,0005 bis 1, vorzugsweise 0,001 bis 0,05 Sekunden.

Im Vergleich zum Stand der Technik liefert diese Variante des erfindungsgemäßen Verfahrens überraschend auf einfacherem und wirtschaftlicherem Wege ein besseres Gesamtergebnis in Bezug auf Ausbeute, Raumzeitausbeute und Reinheit der Endstoffe. Insbesondere ist die Menge an Produkt zum eingesetzten Katalysator sehr viel größer, so daß trotz Verwendung des teuren Edelmetall-Katalysators Silber ein wirtschaftliches Verfahren realisiert werden kann. Der Katalysator ist einfach herzustellen und erlaubt aufgrund der regelmäßigen kugelförmigen Gestalt eine einfache Befüllung des Reaktors. Ein weiterer Vorteil der regelmäßigen Form des Katalysators ist, daß ohne weitere Maßnahmen eine geordnete dichtere Packung im Reaktor erzielt wird und bei Rohrbündelreaktoren jedes Einzelrohr aufgrund der gleichmäßigen Schüttung ähnlichen Druckverlust zeigt. Der in den vielen Röhren eines Rohrbündelreaktors gleiche Druckverlust führt zu gleicher Anströmung der einzelnen Röhren und dadurch offenbar zu einer wesentlichen Verbesserung der Selektivität der Umsetzung. Im Verlauf der Reaktion werden einzelne Rohre nicht stärker belastet, so daß die Standzeit des Katalysators sehr hoch ist und in der Praxis bei mehreren Monaten liegt.

Es war überraschend, daß sich auf diese Weise selbst die instabilen Hydroxyketone, wie Acetoin und deren Ketalderivate unter den drastischen Bedingungen der oxidativen Dehydrierung mit hohen Umsätzen und gleichzeitig Selektivitäten von über 90 % der Theorie zu den entsprechenden Diketonen bzw. den Monoketalen von Diketonen umsetzen lassen. Vorteilhaft beim Einsatz der Hydroxyketale der Formel II ist der direkte Zugang zu diesen Ausgangsverbindungen, die sonst nur über eine mehrstufige Reaktion zu erhalten sind, aus Ketonen.

### Zu Variante b) des erfindungsgemäßen Verfahrens

Auch bei dieser Verfahrensvariante verwendet man als oxidierendes Agens freien Sauerstoff oder auch freien Sauerstoff enthaltende Gase, insbesondere Luft. Sauerstoff und Alkohol II werden zweckmäßig im Molverhältnis von 0,25 bis 0,9, vorzugsweise 0,35 bis 0,70 Mol Sauerstoff pro Mol Alkohol der Formel II verwendet.

Als Katalysator verwendet man elementares Silber und/oder Kupfer in Form von Silberkristallen und/oder Kupferkristallen mit etwa 0,1 bis 2,5 mm Durchmesser. Im allgemeinen verwendet man Mischungen von Silber- und/oder Kupferkristallen mit verschieden großem Durchmesser.

Mit besonderem Vorteil verwendet man Silberkristalle mit verschieden großem Durchmesser. Die Katalysatorkristalle können in einer einzelnen Schicht oder in mehreren übereinander angeordneten Schichten verwendet werden. Im Einschichtkatalysator liegt der gröberkörnige Anteil der Katalysatorkristalle homogen in der Schicht verteilt vor.

Beim Einsatz von 2, 3 oder mehr Katalysatorschichten unterscheiden sich die einzelnen Schichten bezüglich der Korngröße der Katalysatorkristalle und meistens auch im zugehörigen Gewichtsanteil des Gesamtkatalysators. In einem 2-Schichtkatalysator liegt zweckmäßig eine Schicht von Kristallen mit einer Korngröße von 0,1 bis 0,75 mm und eine mit der Korngröße von 0,75 bis 2,5 mm vor.

Ist der Katalysator in 3 Schichten angeordnet, so betragen die Korngrößen der einzelnen Schichten beispielsweise 0,1 bis 0,75 mm, 0,75 bis 1 mm und 1 bis 2,5 mm oder 0,2 bis 0,4 mm, 0,4 bis 0,75 mm und 0,75 bis 1 mm.

Bezüglich der Herstellungsmethoden von Silber wird auf Ullmanns Encyklopädie der technischen Chemie, Band 15, Seiten 636 bis 666, verwiesen. Ebenfalls kann Silber aus entsprechenden Lösungen, z.B. Silbernitratlösungen mit Fällungsmitteln, z.B. mit Hydrazin oder Formaldehyd, ausgefällt oder durch Elektrolyse gewonnen werden.

Das gesamte Katalysatorbett liegt zweckmäßig auf einem Netz aus Silber oder Edelstahl (vorgeglüht). Bei großen Reaktoren mit einem Durchmesser von mehr als 15 cm wird das Netz zweckmäßig vor dem Einbau gewellt.

Die Gesamtschichtdicke des Katalysators beträgt im allgemeinen etwa 5 bis 50, vorzugsweise 10 bis 30 mm.

Zur Durchführung des Verfahrens leitet man im allgemeinen ein Gemisch des aus dem Verdampfungsaggregat kommenden dampfförmigen Alkohols der Formel II mit Sauerstoff oder dem Sauerstoff enthaltenden Gas bei den Reaktionstemperaturen über den Katalysator. Die Temperatur am Katalysator wird zweckmäßig mit Thermoelementen gemessen.

Der bei der Umsetzung herrschende Druck ist nicht kritisch. Die Reaktion wird daher in der Regel bei Normaldruck durchgeführt. Es kann jedoch auch vorteilhaft sein, bei vermindertem Druck zu arbeiten, wenn der zu oxidierende Alkohol der Formel II schwer verdampfbar ist.

Für einige Umsetzungen hat es sich als vorteilhaft erwiesen, auf den Katalysator noch geringe Mengen an Phosphorsalzen, wie Phosphate, oder Polyphosphate durch Aufstreuen oder durch Abscheiden aus Lösungen aufzubringen.

Hierfür werden Alkalimetall- oder Erdalkalimetallphosphate oder -pyrophosphate, wie Na₄P₂O₇, Li₃PO₄, Mg₃(PO₄)₂ oder Ca₃(PO₄)₂ verwendet. Die Menge der eingesetzten Phosphorverbindung liegt bei 0,05 bis 100 mg Phosphor pro Gramm Katalysator.

Mit Hilfe des erfindungsgemäßen Verfahrens ist es möglich, die wegen ihrer besonderen olfaktorischen Eigenschaften begehrten Diketone, wie Diacetyl, 1,2-Cyclo-pentandion, 1,2-Cyclohexandion, 2,3-Pentandion, 3,4-Hexandion und 2,3-Hexandion sowie die Monoketale der 1,2-Cycloalkandione auf einfache Weise in hoher Selektivität bei sehr hohen Umsätzen und Raumzeitausbeuten sowie mit guten sensorischen Eigenschaften herzustellen.

### Beispiel 1

### A) Herstellung von 3,3-Dimethoxy-2-butanol

In einer ungeteilten Elektrolysezelle (Plattenstapelzelle) mit 10 Spalten, 1,45 dm² Fläche pro Elektrode, einer Kohlefilz KFD-2-Anode und einer Graphitplatte MKUS P-10-Kathode (beide von der Firma SGL Carbon, Spacer Polypropenylnetz 0,9 mm) wurde bei Temperaturen von < 20°C, einer Stromstärke von 3,06 A/dm², einer Strommenge von 1,5 F und einem Durchfluß von 45 bis 60 I/(h * Spalt) eine Elektrolytlösung bestehend aus 1000 g 2-Butanon, 45 g KJ, 10 g KOH, 5 g Wasser und 1950 g Methanol elektrochemisch oxidiert.
Das 3,3-Dimethoxy-2-butanol wurde hierbei in einer Selektivität von 70 % der Theorie bei einem Umsatz von 58 % erhalten.

### B) Herstellung von 3,3-Dimethoxy-2-butanon

553 g 3,3-Dimethoxy-2-butanol wurden pro Stunde (h) verdampft und mit Luft (0,6 mol Sauerstoff pro mol Edukt) 500°C an einer aus 3 Schichten bestehenden Schüttung bestehend aus 8 g Silberkörnern mit einem Durchmesser von 0,75 bis 1 mm, 14 g mit einem Durchmesser von 0,4 mm bis 0,75 mm und 6 g mit einem Durchmesser von 0,2 bis 0,4 mm (Schüttvolumen 6 ml) umgesetzt. Die Katalysatorbelastung betrug 20 kg Edukt/kg.h. Es wurden 595 g eines Gemisches erhalten, das aus 82,5 % 3,3-Dimethoxy-2-butanon und 1,2 % Diacetyl bestand. Das entspricht einer Ausbeute an Wertprodukt von 91,34 % der Theorie, bzw. 90,04 % der Theorie an 3,3-Dimethoxy-2-butanon und 1,31 % der Theorie an Diacetyl.

### C) Herstellung von Diacetyl aus 3,3-Dimethoxy-2-butanon

Unter Zusatz von Wasser und/oder verdünnten Mineralsäuren wird das 3,3-Dimethoxy-2-butanon während der anschließenden destillativen Aufarbeitung gespalten. Bei geeigneter Auslegung der Destillationskolonne fällt das Diacetyl in einer Reinheit von > 99 % an.

### Beispiel 2

### Oxidation von 3-Hydroxy-2-butanon zu Diacetyl

14,5 g eines Schalenkatalysators, der 6 Gew.-% Silber als geschlossene Schale aus metallischem Silber auf Steatitkugeln mit einem Durchmesser von 0,2 bis 0,25 mm enthielt, wurden in einem Rohrreaktor mit einem Durchmesser von 11 mm eingebracht, wodurch eine 10 cm hohe Katalysatorschicht gebildet wurde. Durch diese Katalysatorschicht wurden bei den aus der Tabelle ersichtlichen Reaktionstemperaturen Acetoin (enthaltend ca. 12 % Wasser) und Sauerstoff in Form von Luft im aus der Tabelle ersichtlichen Mol-Verhältnis in einer aus der Tabelle ersichtlichen Katalysatorbelastung geleitet.

Die hierbei erzielten Umsätze des Acetoins sowie die erzielten Ausbeuten an Diacetyl pro umgesetztem Acetoin (Selektivitäten) sind in der folgenden Tabelle angegeben.

**Tabelle**

| Beispiel | Temperatur [°C] A = Anfangstemperatur E = Endtemperatur | Mol-Verhältnis Acetoin: ½ O₂ | Kat.-Belastung t/m^{2.}h | Umsatz [%] | Selektivität [%] |
|---|---|---|---|---|---|
| 2a | A 370 | 1:1,10 | 0,52 | 90,2 | 90,0 |
| | E 540 | | | | |
| 2b | A 360 | 1: 1,12 | 0,51 | 91,7 | 92,1 |
| | E 533 | | | | |
| 2c | A 361 | 1:1,09 | 0,52 | 87,8 | 89.3 |
| | E 534 | | | | |
| 2d | A 324 | 1:1,11 | 0,51 | 83,6 | 91,4 |
| | E 537 | | | | |
| 2e | A 329 | 1:1,12 | 0,51 | 76,9 | 95,7 |
| | E 518 | | | | |
| 2f | A 305 | 1:1,12 | 0,51 | 79,8 | 91,4 |
| | E 543 | | | | |
| 2g | A 289 | 1:1,10 | 0,52 | 85,1 | 89.3 |
| | E 572 | | | | |
| 2h | A 408 | 1:1,13 | 0,51 | 66,1 | 93,9 |
| | E 499 | | | | |
| 2i | A 427 | 1:1,13 | 0,50 | 65,3 | 96,1 |
| | E 487 | | | | |
| 2k | A 424 | 1:1,10 | 0,52 | 47,9 | 97,6 |
| | E 458 | | | | |

### Beispiel 3

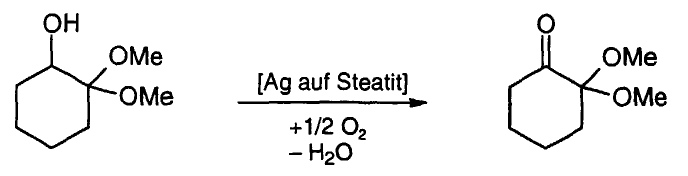

55 g (0,34 mol) 2,2-Dimethoxy-cyclohexanol wurden pro h verdampft und mit Luft (0,65 mol Sauerstoff pro mol Edukt) bei 500°C über einen mit 17 g Silber auf Steatitkügelchen (6 % Ag) gefüllten Kurzrohrreaktor (Länge: 10 cm; Durchmesser: 11 mm) geleitet. Die Katalysatorbelastung betrug 3,2 kg Edukt/(kg·h). Es wurden pro h 54 g eines Gemisches aus 88 % 2,2-Dimethoxy-cyclohexanon und 6 % Cyclohexan-1,2-dion erhalten. Der Umsatz an Edukt war vollständig.

Das entspricht einer Ausbeute von 94,5 % der Theorie an Wertprodukt, bzw. einer Ausbeute von 88,5 % an 2,2-Dimethoxy-cyclohexanon und 6 % an Cyclohexan-1,2-dion.

## Patentansprüche

1. Verfahren zur Herstellung von Carbonylverbindungen der allgemeinen Formel I 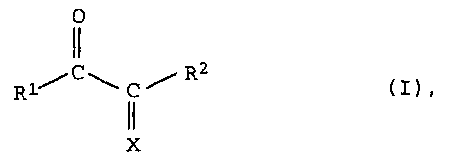 in der R¹ und R² für einen verzweigten oder unverzweigten aliphatischen, cycloaliphatischen, aromatischaliphatischen und cycloaliphatischaliphatischen Rest mit 1 bis 10 C-Atomen stehen, oder aber
R¹ und R² zusammen für eine gegebenenfalls durch niedere Alkylgruppen substituierte Alkylengruppe mit 3 bis 10 C-Atomen steht,
und x für = O oder 2 Alkoxygruppen -OR³ steht, wobei R³ für einen gesättigten oder ungesättigten, verzweigten oder unverzweigten aliphatischen Rest mit 1 bis 4 C-Atomen steht, oder aber die beiden R³ zusammen für eine gegebenenfalls durch niedere Alkylgruppen substituierte Alkylengruppe mit 3 bis 6 C-Atomen stehen,
dadurch gekennzeichnet, daß man Alkohole der allgemeinen Formel II 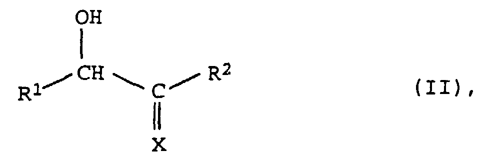 in der R¹ bis R³ und X die oben angegebene Bedeutung haben, mit Sauerstoff in der Gasphase
a) bei Temperaturen von 270 bis 600°C an Silberschalenkatalysatoren, die eine abriebfeste Schale von metallischem Silber auf einem Kern aus inertem Trägermaterial enthalten, oder aber
b) bei Temperaturen von 450 bis 750°C an Silberkristallen und/oder Kupferkristallen mit einer Korngröße von 0,1 bis 2,5 mm während einer Verweilzeit von maximal 0,1 Sekunden oxidiert.

2. Verfahren gemäß Anspruch 1, dadurch gekennzeichnet, daß man Alkohole der allgemeinen Formel II, in der X für O steht, und R¹ und R² die obenangegebene Bedeutung haben, an Silberschalenkatalysatoren oxidiert, die elementares Silber auf kugelförmigem Inertmaterial enthalten.

3. Verfahren gemäß Anspruch 2, dadurch gekennzeichnet, daß man Alkohole der allgemeinen Formel II, in der X für = O steht, und R¹ und R² die obenangegebene Bedeutung haben, in einem Reaktionsrohr oder einem Reaktionsrohrbündel an Silberschalenkatalysatoren oxidiert, die elementares Silber auf Steatit-Kügelchen enthält.

4. Verfahren gemäß Anspruch 1, dadurch gekennzeichnet, daß man Alkohole der allgemeinen Formel II, in der X für zwei Alkoxygrupppen mit je 1 bis 4 C-Atomen steht, an Silberkristallen mit einer Korngröße von 0,1 bis 2,5 mm mit Sauerstoff oxidiert.

5. Verfahren gemäß Anspruch 1, dadurch gekennzeichnet, daß man die bei der Oxidation von Alkoholen der allgemeinen Formel II, in der X für zwei Alkoxygruppen steht, erhaltenen Carbonylverbindungen der allgemeinen Formel I, in der X für zwei Alkoxy-Gruppen steht, anschließend in an sich bekannter Weise in Carbonylverbindungen der allgemeinen Formel I, in der X für = O steht, hydrolysiert.

6. Verfahren gemäß Anspruch 1, dadurch gekennzeichnet, daß man als Ausgangsverbindungen Alkohole der allgemeinen Formel II, in der X für zwei Alkoxygruppen steht, verwendet, die durch elektrochemische Oxidation von Ketonen der allgemeinen Formel III 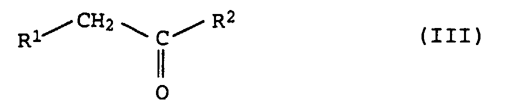 in Gegenwart von C₁- bis C₄-Alkanolen, Wasser und Hilfselektrolyten erhalten worden sind.

7. Verfahren zur Herstellung von Carbonylverbindungen der allgemeinen Formel I 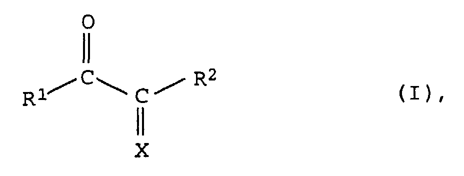 in der R¹ und R² für eine Methylgruppe stehen, oder aber R¹ und R² zusammen für eine ggf. durch niedere Alkylgruppen substituierte Alkylengruppe mit 3 bis 10 C-Atomen steht,
und X für = O steht, dadurch gekennzeichnet, daß man
A. Alkohole der allgemeinen Formel II 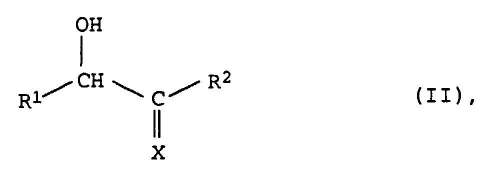
in der R¹ bis R² die oben angegebene Bedeutung haben, und X für zwei Alkoxygruppen -OR³ steht, in der R³ eine Alkylgruppe mit 1 bis 4 C-Atomen bedeutet, oder aber die beiden R³ zusammen für eine gegebenenfalls durch niedere Alkylgruppen substituierte Alkylengruppe mit 3 bis 6 C-Atomen stehen, verwendet, die durch elektrochemische Oxidation von Ketonen der allgemeinen Formel III 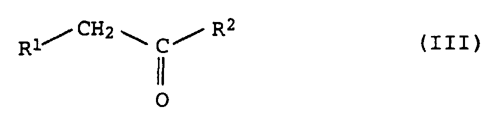 in Gegenwart von C₁- bis C₄-Alkanolen, Wasser und Hilfselektrolyten hergestellt worden sind,
B. die erhaltenen Alkohole der allgemeinen Formel II mit Sauerstoff in der Gasphase
a) bei Temperaturen von 270 bis 600°C an Silber-Schalenkatalysatoren, die eine abriebfeste Schale von metallischem Silber auf einem Kern aus inertem Trägermaterial enthalten, oxidiert oder aber
b) bei Temperaturen von 450 bis 750°C an Silberkristallen mit einer Korngröße von 0,1 bis 2,5 mm während einer Verweilzeit von maximal 0,1 Sekunden oxidiert und
C. die dabei erhaltenen Carbonylverbindungen der allgemeinen Formel I, in der X für zwei Alkoxygruppen steht, anschließend in an sich bekannter Weise in Carbonylverbindungen der allgemeinen Formel I, in der X für = O steht, hydrolysiert.
